# EUROPÄISCHE PATENTSCHRIFT

(11) **EP 0 902 021 B1**
(45) Veröffentlichungstag und Bekanntmachung des Hinweises auf die Patenterteilung: **06.07.2005**
(21) Anmeldenummer: 98114956.0
(22) Anmeldetag: 08.08.1998
(51) Int. Cl.: C07D 255/02, C07D 251/04, C11D 1/40, C11D 3/39, C11D 3/28

(54) **Cyclische Polyaminsalze**
Cyclic polyamine salts
Sels de polyamines cycliques

(30) Priorität: 02.09.1997 DE 19738273
(43) Veröffentlichungstag der Anmeldung: 17.03.1999
(73) Patentinhaber: Clariant GmbH, 65929 Frankfurt am Main (DE)
(72) Erfinder: Seebach, Michael, Dr., 65795 Hattersheim (DE); Reinhardt, Gerd, Dr., 65779 Kelkheim (DE)

(56) Entgegenhaltungen:
- EP-A- 0 458 397
- EP-A- 0 458 398
- EP-A- 0 549 271
- EP-A- 0 549 272
- DE-A- 19 629 159

## Beschreibung

Die vorliegende Erfindung betrifft cyclische Polyaminsalze als Aktivatoren beziehungsweise Katalysatoren für Persauerstoffverbindungen und Wasch- und Reinigungsmittel, die diese Verbindungen enthalten.

Peroxidbleichmittel in wäßrigen Wasch- und Reinigungsmitteln, insbesondere für die Textilwäsche, sind seit langem bekannt. Solche Mittel entfalten ihre Wirkung, beispielsweise die Entfernung von Tee-, Obst- oder Rotwein-Verschmutzungen von Textilien, am besten bei Temperaturen von 60 bis 100°C; bei Temperaturen unterhalb 60°C ist ihre Wirkung stark eingeschränkt.

Es ist bekannt, daß viele Übergangsmetallionen die Zersetzung von Wasserstoffperoxid oder Wasserstoffperoxid freisetzenden Verbindungen wie Natriumperborat oder Natriumpercarbonat bei niedrigeren Temperaturen katalysieren. Es ist auch vorgeschlagen worden, daß Übergangsmetallsalze zusammen mit chelatisierenden Verbindungen zur Aktivierung von Peroxidbleichmitteln gerade bei niedrigen Temperaturen eingesetzt werden können. Die als Bleichkatalysatoren in Waschmitteln verwendeten Übergangsmetallverbindungen sollten die Zersetzung der Peroxide über nicht bleichende Zerfallsreaktionen nicht übermäßig fördern und sollten zudem hydrolyse- und oxidationsstabil sein. Die meisten bislang bekannten Peroxidbleiche-Katalysatoren enthalten Kobalt oder Mangan als Übergangsmetall. Aus ökologischen Gründen erscheint die Verwendung von kobalthaltigen Verbindungen jedoch nicht akzeptabel. In einer Reihe von Patenten wird die Verwendung des unbedenklichen Übergangsmetalles Mangan beschrieben. Ein Teil dieser Anwendungen beruht auf der katalytischen Aktivität freier Manganionen und erfüllt nicht das Kriterium der Hydrolysestabilität und führt zudem zu Faserschäden. US-Patent 4,728,455 beschreibt die Verwendung von Mn-(III)-gluconat als Peroxidbleiche-Katalysator mit hoher Hydrolyse- und Oxidationsstabilität. Das katalytische System erfordert jedoch große Mengen an Ligand, bezogen auf Manganionen, und ist unzulänglich für die Niedrigtemperaturbleiche bei 20 bis 40°C. In EP-A-458 397, EP-A-458 398 und EP-A 549 272 werden Mangan-Komplexe mit cyclischen Polyaminen als Bleichkatalysatoren beschrieben. Als Liganden werden hierbei hauptsächlich aliphatische Makrocyclen mit 3 oder 4 Heteroatomen, meist Stickstoffatomen, die an der freien Bindung eine Methylgruppe tragen oder unsubstituiert sind, verwendet. Die beschriebenen Mangan-Komplexe erfüllen die Forderung nach Stabilität und sind außerordentlich aktiv schon bei niedrigen Temperaturen und gegenüber einer großen Anzahl unterschiedlicher Schmutzarten. Ein schwerwiegendes Problem stellen jedoch die starken Faserschäden dar, die mit diesen Verbindungen bei der Wäsche von Baumwolle beobachtet werden.

Die Verwendung cyclischer Amine als Bleich-Katalysatoren ist in EP-A-549 271 beschrieben. In diesem Dokument wird jedoch ausdrücklich darauf hingewiesen, daß diese cyclischen Amine nur gemeinsam mit einer Verbindung, die Eisen oder Mangan abgibt, wirksam sind. Diese Quelle für Eisen oder Mangan kann ein entsprechendes Salz sein, das zusätzlich zu dem cyclischen Amin in dem Wasch- und Reinigungsmittel vorhanden sein muß, oder das Eisen bzw. Mangan wird aus den zu behandelnden Materialien heraus aufgenommen. In jedem Fall müssen Metall-Ionen vorhanden sein, denn ein Abfangen dieser Metall-Ionen durch Zugabe eines Sequestrierungsmittels verschlechtert gemäß den Angaben dieses Dokuments die Bleichwirkung.

Im Gegensatz zu der Lehre gemäß EP-A-549 271 wurde nun gefunden, daß sich derartige cyclische Polyamine in Salzform sehr wohl ohne Zugabe einer Quelle für Schwermetall-Ionen als Aktivatoren bzw. Katalysatoren eignen.

Gegenstand der Erfindung sind cyclische Polyamin-Salze der Formel 1 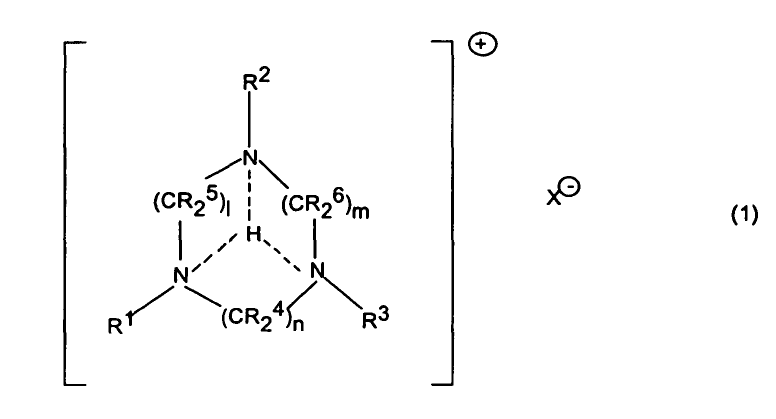 worin R¹, R², R³, R⁴, R⁵ und R⁶ unabhängig voneinander Wasserstoff oder C₁-C₄-Alkyl und l, m und n unabhängig voneinander die Zahl 2 und X ein Anion bedeutet.

In obiger Formel bedeuten R¹, R², R³, R⁴, R⁵ und R⁶ vorzugsweise Wasserstoff oder Methyl. Bevorzugt bedeuten R¹, R² und R³ C₁-C₄-Alkyl, insbesondere Methyl und R⁴, R⁵ und R⁶ Wasserstoff. X ist die äquivalente Menge eines Anions beispielsweise Cl⁻, Br⁻, J⁻, NO₃⁻, ClO₄⁻, NCS⁻, PF₆⁻, RSO₄⁻, RCOO⁻, BPh₄⁻, CF₃SO₃⁻, RSO₃⁻, wobei R C₁-C₄-Alkyl und Ph Phenyl bedeutet.

Beispiele für cyclische Polyamine, aus denen durch Reaktion mit geeigneten Säuren die entsprechenden Salze der Formel (1) hergestellt werden können, finden sich in der folgenden Aufstellung:
1,4,7-Triazacyclononan, 1,4,7-Triazacyclododecan, 1,4,8-Triazacyclododecan,
1,4,7-Trimethyl-1,4,7-triazacyclononan, 1,4,7-Trimethyl-1,4,7-triazacyclododecan.

Diese Verbindungen können am Stickstoffatom und/oder an der CH-Gruppe weitere Substituenten tragen. Bevorzugt sind folgende cyclischen Polyamine:
1,4,7-Triazacyclononan (TACN), 1,4,7-Trimethyl-1,4,7-triazacyclononan(1,4,7-Me₃TACN), 2-Methyl-1,4,7-triazacyclononan (2-MeTACN), 1,4-Dimethyl-1,4,7-triazacyclononan (1,4-Me₂TACN), 1,2,4,7-Tetramethyl-1,4,7-triazacyclononan (1,2,4,7-Me₄TACN), 1,2,2,4,7-Pentamethyl-1,4,7-triazacyclononan(1,2,2,4,7-Me₅TACN), 2-Benzyl-1,4,7-trimethyl-1,4,7-triazacyclononan, 2-Decyl-1,4,7-trimethyl-1,4,7-triazacyclononan.

Die erwähnten cyclischen Polyamine können nach Methoden hergestellt werden, wie sie zum Beispiel von K. Wieghardt et al. beschrieben sind in Inorganic Chemistry 1982, 21, 3086 ff. oder den dort zitierten Literaturstellen sowie in "Macrocyclic Chemistry" von Dietrich, Viout, Lehn, Weinheim 1993.

Aus diesen cyclischen Polyaminen werden nach an sich bekannten Verfahren die Salze der Formel (1) durch Umsetzung mit entsprechenden Säuren erhalten.

Die erfindungsgemäßen protonierten cyclischen Polyamine eignen sich in hervorragender Weise als Bleichkatalysatoren in Wasch- und Reinigungsmitteln. Besonders bevorzugt sind hier Textilwaschmittel in Form von Pulverwaschmitteln oder als flüssige Formulierungen und Geschirreinigungsmittel. Ein Vorteil der erfindungsgemäßen Bleichkatalysatoren ist dabei ihre Stabilität gegenüber Hydrolyse und Oxidation. Sie verbessern in Waschmittelformulierungen nicht nur die Bleichwirkung von Wasserstoffperoxid, sondern auch die von organischen und anorganischen Peroxysäure-Verbindungen.

Als weitere anwendungstechnische Vorteile sind zu nennen ihre universelle Wirksamkeit bei der Entfernung aller Arten von Verschmutzungen, sowohl hydrophiler als auch hydrophober Natur, und ihre Verträglichkeit mit den üblichen Waschmittelenzymen wie Proteasen, Cellulasen, Lipasen, Amylasen oder Oxidasen.

Gegenstand der vorliegenden Erfindung ist demgemäß ein Verfahren zum Bleichen von verschmutzten Substraten, wobei man das verschmutzte Substrat in wäßriger Bleichflotte mit Peroxyverbindungen und einer wirksamen Menge eines oder mehrerer protonierter cyclischer Polyamine der Formel (1) als Bleichkatalysatoren in Kontakt bringt. Dabei enthält die wäßrige Bleichflotte diese protonierten Polyamine, bezogen auf das Gewicht der Bleichflotte, vorzugsweise in einer Menge von 0,001 bis 500 ppm, insbesondere von 0,01 bis 80 ppm, vor allem von 0,1 bis 30 ppm (ppm bedeutet "parts per million", bezogen auf das Gewicht). Wäßrige Wasch- und Bleichflotten, die Peroxyverbindungen und die erfindungsgemäßen Bleichkatalysatoren enthalten, sind im neutralen und im alkalischen pH-Bereich, also von ca. pH 7 bis pH 14, wirksam. Ein Wirkungsoptimum liegt bei pH 8 bis pH 11.

Gegenstand der vorliegenden Erfindung sind weiterhin Wasch- und Reinigungsmittel-Formulierungen, enthaltend neben den üblichen Bestandteilen übliche Mengen an Peroxyverbindungen und eine wirksame Menge eines oder mehrerer protonierter cyclischer Polyamine der Formel (1) als Bleichkatalysatoren. Als wirksame Menge der Bleichkatalysatoren sind üblicherweise Mengen von 0,0001 Gew.-% bis 2 Gew.-% insbesondere von 0,001 Gew.-% bis 0,8 Gew.-%, vor allem von 0,005 Gew.-.% bis 0,5 Gew.-%, bezogen auf das Gewicht der Formulierungen, ausreichend. Diese Mengen können je nach landesüblichen Gepflogenheiten in den Waschmittelzusammensetzungen leicht schwanken.

Das erfindungsgemäße Verfahren zum Bleichen von verschmutzten Substraten sowie die erfindungsgemäßen Wasch- und Reinigungsmittelformulierungen zeichnen sich besonders dadurch aus, daß durch den Bleichvorgang keine Faserschädigungen verursacht werden. Außerdem sind die erfindungsgemäßen Katalysatoren metallfreie Verbindungen, was aus ökologischen Gründen von Vorteil ist.

Peroxyverbindungen, die zusammen mit den erfindungsgemäßen Bleichkatalysatoren verwendet werden können, umfassen Wasserstoffperoxid, Wasserstoffperoxid freisetzende Verbindungen, Wasserstoffperoxid erzeugende Systeme, Peroxysäuren und ihre Salze und Peroxysäure-Vorstufen, sowie Mischungen daraus. Als Wasserstoffperoxid-Quellen sind beispielsweise Alkalimetallperoxide, Harnstoff-H₂O₂-Komplexe und anorganische Persalzverbindungen, wie Alkalimetallperborate, -percarbonate, -perphosphate, -persilicate und -persulfate bekannt. Gemische aus zwei oder mehreren solcher Verbindungen können ebenfalls verwendet werden. Von besonderer Bedeutung sind Natriumperborat-monohydrat, -Tetrahydrat und Natriumpercarbonat. Natriumpercarbonat ist auch aus ökologischen Gründen zu bevorzugen. Eine weitere Klasse von Peroxid-Bleichmitteln sind die Alkylhydroxyperoxide. Beispiele hierfür sind Cumolhydroperoxid und Tert-butyl-hydroperoxid.

Die genannten Bleichmittel können in Kombination mit Peroxysäure-Vorstufen eingesetzt werden. Beispiele für Peroxy-Vorstufen mit quaternären Ammonium-Strukturen sind 2-(N,N,N-Trimethylammonium)ethyl-4-sulfophenylcarbonat (SPCC), N-Octyl-N,N-dimethyl-N-10-carbophenoxydecylammoniumchlorid (ODC), 3-(N,N,N-Trimethylammonium)propyl-natrium-4-sulfophenylcarboxylat und N,N,N-Trimethylammonium-toluyloxybenzolsulfonat.

Bevorzugte Klassen von Bleichmittel-Vorstufen, d.h. Peroxysäure-Vorstufen, sind neben den oben genannten quaternären Ammoniumsalzen Ester einschließlich der Acylphenolsulfonate und der Acylalkylphenolsulfonate sowie Acylamide.

Von besonderem Interesse sind hierbei die in der Praxis gern eingesetzten, oft auch als Bleichaktivatoren bezeichneten Verbindungen Natrium-4-benzoyloxybenzolsulfat (SBOBS), N,N,N',N'-Tetraacetylethylendiamin (TAED), Natrium-1-methylbenzoyloxy-benzol-4-sulfonat, Natrium-4-methyl-3-benzoyloxy-benzoat, Natriumnonanoylbenzolsulfonat (SNOBS), Natrium-3,5,5-trimethylhexanoyloxybenzolsulfonat (STHOBS), 2-Phenyl-benz-(4H)1,3-oxazin-4-on, Glucosepentaacetat und Tetraacetylxylose. Weitere verwendbare Vorstufen sind die sogenannten Sulfonimide.

Auch aliphatische und aromatische Mono- oder Dicarbonsäuren eignen sich als Peroxyverbindungen. Beispiele hierfür sind Peroxy-α-naphthoesäure, Peroxylaurinsäure, Peroxystearinsäure, N,N-Phthaloylaminoperoxycapronsäure (PAP), 1,12-Diperoxydodecandisäure, 1,9-Diperoxyazelainsäure, Diperoxysebacinsaure, Diperoxyisophthalsäure, 2-Decyldiperoxybutan-1,4-disaure und 4,4-Sulfonylbisperoxybenzoesäure.

Weiterhin eignen sich als Peroxyvberbindungen anorganische Peroxysäure-Salze, z.B. Kaliummonopersulfat. Die erfindungsgemäßen Waschmittel-Formulierungen enthalten üblicherweise 1 bis 30 Gew.-%, insbesondere 2 bis 25 Gew.-% an Peroxyverbindungen. Persäuren können in etwas kleineren Mengen verwendet werden, beispielsweise von 1 bis 15 Gew.-%, bevorzugt 2 bis 10 Gew.-%. Persäurevorstufen können in Kombination mit einer Peroxyverbindung in ähnlicher Konzentration wie die Persäuren eingesetzt werden, z.B. von 1 bis 15 Gew.-%, vorzugsweise von 2 bis 10 Gew.-%.

Die erfindungsgemäßen Waschmittelformulierungen enthalten in der Regel weiterhin die hierfür üblichen Bestandteile in den hierfür üblichen Mengen, d.h. insbesondere oberflächenaktive Substanzen und Gerüst- oder Builder-Substanzen. Die erfindungsgemäßen Bleichkatalysatoren sind mit diesen Bestandteilen der Formulierungen sowie mit weiteren gegebenenfalls enthaltenen Hilfsmitteln weitgehend verträglich.
Die Art und die Menge von oberflächenaktiven Substanzen (Tensiden), Gerüst-. oder Builder-Substanzen und Hilfsmitteln für Waschmittel-Formulierungen sind dem Fachmann geläufig und brauchen hier deshalb nicht weiter ausgeführt werden.

Bei anwendungstechnischen Tests zeigten marktübliche Kompaktwaschmittelformulierungen, die die erfindungsgemäßen Bleichkatalysatoren in den angegebenen Mengen enthielten, eine beträchtliche Erhöhung der Bleichwirkung, bei mit Tee, Blut, Gras, Rotwein und anderen Testanschmutzungen verschmutztem Textilgewebe.

### Beispiele

Die Bleichversuche wurden mit Natriumperborat-Monohydrat oder Natriumpercarbonat an Baumwollstoff mit Standard-Anschmutzungen durchgeführt. Alle Experimente wurden entweder in der Waschmaschine oder im Linitest-Gerät durchgeführt.
Für die Versuche wurde entmineralisiertes Wasser durch Zugabe von Calciumchlorid p.A. auf eine Härte von 15°dH eingestellt.

Experimentelle Bedingungen sind bei den einzelnen Beispielen angegeben. Die Remission R wurde mit Hilfe eines Elrephometers der Fa. Datacolor vor und nach dem Waschvorgang ermittelt. Die Differenz ΔR dieser Werte stellt ein Maß für den Bleicheffekt dar.

Das verwendete Grundwaschmittel (WMP) setzt sich wie folgt zusammen:

| Komponenten | Teile |
|---|---|
| lineares Alkylbenzolsulfonat (Cₘᵢₜₜₑₗ = 11,5) | 7,5 |
| C₁₂₋₁₈-Alkohol *EO₇ | 4,0 |
| Seife (65 % C₁₂₋₁₈, 35 % C₂₀₋₂₂) | 2,8 |
| Zeolith A | 25,0 |
| Natriumcarbonat | 9,1 |
| Na-Salz eines Copolymerisats aus Acryl- und Maleinsäure (CP₅) | 4,0 |
| Natriumsilikat (SiO₂:NaO₂ = 3,32:1) | 2,6 |
| Carboxymethylcellulose | 1,0 |
| EDTA | 0,2 |
| Stilbenaufheller | 0,2 |
| Natriumsulfat, Wasser, Schauminhibitor | 20,1 |
| Enzymprills Protease (Aktivität 300.00) | 0,5 |

Zur Ausprüfung der Katalysatoren wurde in allen Fällen das oben stehende Grundwaschmittel WMP in Verbindung mit Natriumperborat-Monohydrat PB*1 oder Natriumpercarbonat SPC verwendet.

### Beispiel 1:

Dieses Beispiel demonstriert den Effekt der Bleichwirkung des Hydrogensulfatsalzes von 1,4,7-Me₃TACN im Vergleich zum katalysatorfreien Grundwaschmittel (WMP) sowie einem Waschmittel, das den Katalysator
[Mn₂(µ-O)₃(1,4,7-Me₃TACN)₂](PF₆)₂ (A), beschrieben im Patent EP-A 458 397, enthält. Die Versuche wurden in der Waschmaschine (nur Hauptwäsche) durchgeführt. Die Einsatzmengen betrugen 75 g Grundwaschmittel WMP, 10 g Natriumpercarbonat SPC, 60 mg Katalysator A (gemäß EP-A 549 271) bzw. 150 mg 1,4,7-Me₃HTACN-Hydrogensulfat (erfindungsgemäß).

| | | Remission ΔR in [%] | |
|---|---|---|---|
| Anschmutzung | WMP+SPC | 1,4,7-Me₃HTACN-Hydrogensulfat | [Mn₂(µ-O)₃(1,4,7-Me₃TACN)₂](PF₆)₂ A |
| EMPA-Blut | 63,5 | 73,8 | 63,9 |
| EMPA-Cacao | 57,4 | 59,7 | 56,7 |
| WFK-Make up | 54,6 | 58,2 | 58,1 |
| EMPA-Rotwein | 65,3 | 76,9 | 75,6 |
| WFK-Tee (BC-1) | 51,3 | 59,2 | 59,8 |
| WFK-Johannisbeersaft | 29,7 | 37,1 | 38,8 |
| WFK-Kirschsaft | 58,5 | 68,2 | 68,6 |
| WFK-Gras | 63,2 | 68,4 | 68,4 |
| WFK-Curry | 63,7 | 64,6 | 65,9 |

Die Resultate zeigen, daß die Wirkung des Grundwaschmittels (WMP) durch Zugabe des Salzes 1,4,7-Me₃HTACN-Hydrogensulfat merklich verbessert wird. Das Salz ist in seiner katalytischen Wirkung somit mit dem Katalysator A vergleichbar. Die Dosierung des Katalysators A darf die oben angegebene Menge nicht überschreiten, da es andernfalls durch die intensive Eigenfarbe des Komplexes zu merklichen Gewebeanfärbungen kommen kann.
Der Sachverhalt wird aus Beispiel 2 deutlich.

### Beispiel 2:

Das folgende Beispiel zeigt die Abhängigkeit der Bleichwirkung von der Katalysatorkonzentration. Die Versuche wurden im Linitest-Gerät bei einer Waschdauer von 30 Minuten durchgeführt. Die Einsatzmengen betrugen 1,5 g/l Grundwaschmittel WMP und 0,5 g/l Natriumperborat-Monohydrat. Als Testgewebe wurde WFK-BW-Tee (BC-1) verwendet.

| | Remission ΔR in [%] | |
|---|---|---|
| Katalysatorkonzentration in [mg/l] | 1,4,7-Me₃HTACN-Hydrogensulfat | [Mn₂(µ-O)₃(1,4,7-Me₃TACN)₂](PF₆)₂ A |
| 0 | 52,0 | 52,0 |
| 5 | 57,5 | 58,4 |
| 10 | 57,9 | 56,0 |
| 20 | 57,5 | 54,2 |
| 30 | 57,4 | 53,5 |

Die vorstehende Tabelle zeigt, daß die Bleichleistung von 1,4,7-Me₃HTACN-Hydrogensulfat bis zu einer Konzentration von 10 mg/l ansteigt und bei weiterer Konzentrationserhöhung konstant bleibt. Dies steht im Gegensatz zu vielen Metallkomplexen, z.B. der Mangankomplex A, die als Bleichkatalysator eingesetzt werden können. Aufgrund ihrer starken Eigenfarbe kommt es bei Überdosierung häufig zu Anfärbungen.

### Beispiel 3:

Dieses Beispiel demonstriert die Abhängigkeit der Bleichleistung von der Waschtemperatur. Die Versuche wurden im Linitest-Gerät bei einer Waschdauer von 30 Minuten durchgeführt. Die Einsatzmengen betrugen 1,5 g/l Grundwaschmittel WMP und 0,5 g/l Natriumperborat-Monohydrat PB'1 sowie 5 mg/l Katalysator. Als Testgewebe wurde WFK-BW-Tee (BC-1) verwendet.

| | Remission ΔR in [%] | |
|---|---|---|
| Temperatur in [°C] | WMP + PB*1 | 1,4,7-Me₃HTACN-Hydrogensulfat |
| 20 | 45,7 | 47,8 |
| 40 | 53,0 | 57,5 |
| 60 | 58,8 | 66,3 |
| 80 | 70,7 | 78,3 |

Im gesamten Temperaturbereich liegt die Bleichleistung des katalysatorhaltigen Waschmittels höher als ohne Katalysator. In beiden Fällen ist eine Zunahme mit steigender Temperatur zu beobachten.

### Beispiel 4:

Hier wird gezeigt, wie die Bleichwirkung vom pH-Wert der Waschlauge abhängt. Die Versuche wurden im Linitest-Gerät bei einer Waschdauer von 30 Minuten und einer Waschtemperatur von 23°C durchgeführt. Die Einsatzmengen betrugen 2 g/l Grundwaschmittel WMP und 0,5 g/l Natriumperborat-Monohydrat. Als Testgewebe wurde WFK-BW-Tee (BC-1) verwendet.

| | Remission ΔR in [%] |
|---|---|
| pH-Wert | 1,4,7-Me₃HTACN-Hydrogensulfat |
| 7 | 44,9 |
| 8 | 45,3 |
| 9 | 45,3 |
| 10 | 46,6 |
| 11 | 49,2 |
| 12 | 48,5 |

Das pH-Wert-Optimum wird bei 11 erreicht. Die geprüfte Verbindung ist somit für den Einsatz in marktüblichen Waschmitteln tauglich.

### Beispiel 5:

Waschmittelformulierungen mit Bleichsystemen verursachen häufig Farbschäden an gefärbten Textilien. Das vorliegende Beispiel zeigt das Ausmaß der Farbschäden nach fünf Waschvorgängen. Die Versuche wurden im Linitest-Gerät bei einer Waschdauer von 30 Minuten durchgeführt. Die Einsatzmengen betrugen 1,5 g/l Grundwaschmittel WMP und 0,5 g/l Natriumperborat-Monohydrat PB*1. Als Testgewebe wurde Baumwollstoff mit Remazol Schwarz B als Anschmutzung verwendet.

| Waschmittel | Remission ΔR in [%] |
|---|---|
| WMP + PB*1 | 11,6 |
| 1,4,7-Me₃HTACN-Hydrogensulfat | 12,1 |
| [Mn₂(µ-O)₃(1,4,7-Me₃TACN)₂](PF₆)₂ A | 17,0 |

Das erfindungsgemäße Salz verursacht demnach ebenso wie das katalysatorfreie Waschmittel nur geringe Farbschäden. Im Unterschied hierzu werden durch den Katalysator A deutlich größere Farbschäden verursacht.

### Beispiel 6:

Dieses Beispiel beschreibt die durch Katalysatoren hervorgerufenen Faserschädigungen. Als Maß für die Faserschäden wird der Depolymerisationsgrad (DP) von Baumwolle angeführt. In Abwesenheit von Katalysatoren werden DP-Werte um 2000 gefunden. Diese entsprechen geringer Faserschädigung. Niedrigere Werte stehen für größere Faserschädigung. Nachfolgende Tabelle zeigt die DP-Werte von katalysatorhaltigen Waschmittelformulierungen im Vergleich zum katalysatorfreien Grundwaschmittel. Zur Ermittlung der DP-Werte wurde eine fünffache Wäsche bei einer Katalysatorkonzentration von 2000 ppm durchgeführt.

| Waschmittel | Depolymerisationsgrad (DP) |
|---|---|
| WMP + TAED + SPC | 1969 |
| WMP + TAED + SPC + 1,4,7-Me₃HTACN-Hydrogensulfat | 1954 |
| WMP + TAED + SPC + [Mn₂(µ-O)₃(1,4,7-Me₃TACN)₂](PF₆)₂ A | 369 |

Die Tabelle macht deutlich, daß bei Zugabe des erfindungsgemäßen Bleichkatalysators zum Grundwaschmittel nur geringe zusätzliche Faserschäden beobachtet werden. Im Unterschied hierzu führt die Zugabe von metallhaltigen Bleichkatalysatoren, wie des Mangankomplexes A, zu erheblichen Faserschädigungen.

## Patentansprüche

1. Cyclische Polyamin-Salze der Formel (1) 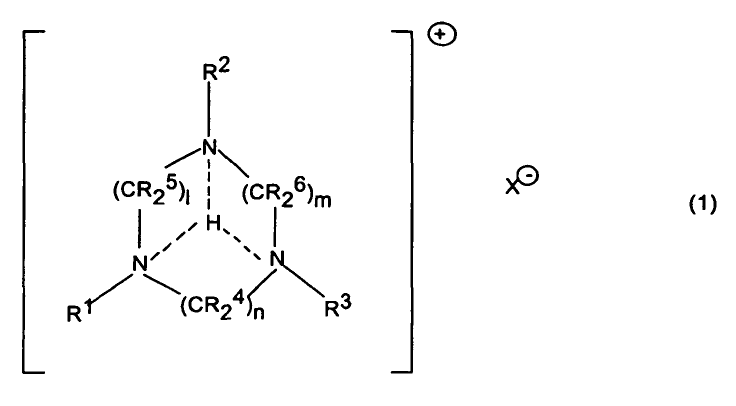 worin R¹, R², R³, R⁴, R⁵ und R⁶ unabhängig voneinander Wasserstoff oder C₁-C₄-Alkyl und l, m und n unabhängig voneinander die Zahl 2 und X ein Anion bedeutet.

2. Cyclische Polyamin-Salze nach Formel (1) gemäß Anspruch 1, wobei R¹, R², und R³ C₁-C₄-Alkyl, R⁴, R⁵ und R⁶ Wasserstoff und l, m und n 2 bedeuten.

3. Cyclische Polyamin-Salze nach Anspruch 1 oder 2, wobei R¹, R², R³ , R⁴, R⁵ und R⁶ Wasserstoff oder Methyl bedeuten.

4. Cyclische Polyamin-Salze nach Anspruch 3, wobei R¹, R² und R³ Methyl bedeuten.

5. Cyclische Polyamin-Salze nach Ansprüchen 1 bis 5, wobei X Cl⁻, Br⁻, J⁻, NO₃⁻, ClO₄⁻, NCS⁻, PF₆⁻, RSO₄⁻, RCOO⁻, BPh₄⁻, CF₃SO₃⁻, RSO₃⁻, R C₁-C₄-Alkyl und Ph Phenyl bedeutet.

6. Verfahren zur Herstellung von cyclischen Polyaminsalzen der Formel 1 nach Anspruch 1, **dadurch gekennzeichnet, daß** man ein cyclisches Polyamin der Formel 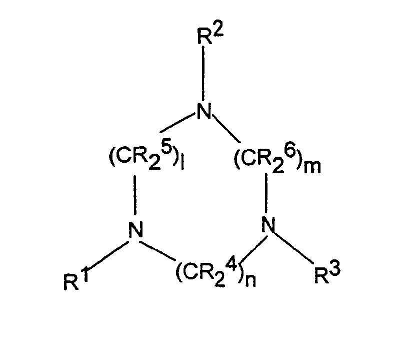 mit einer Säure der Formel H - X umsetzt, wobei R¹, R², R³, R⁴, R⁵, R⁶, l, m, n und X die in Anspruch 1 angegebenen Bedeutungen haben.

7. Wasch- und Reinigungsmittel, enthaltend eine Peroxyverbindung, **dadurch gekennzeichnet, daß** das Wasch- und Reinigungsmittel zusätzlich ein cyclisches Polyamin-Salz der Formel 1 nach Anspruch 1 enthält.

## Claims

1. A cyclic polyamine salt of the formula (1) 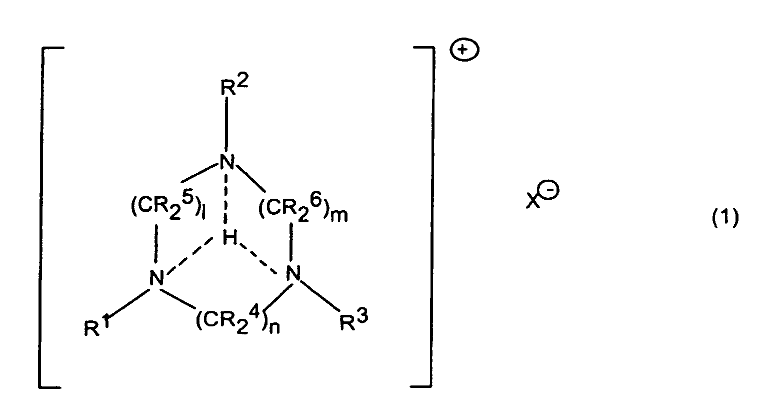 in which R¹, R², R³, R⁴, R⁵ and R⁶ independently of one another are hydrogen or C₁-C₄-alkyl, and I, m and n independently of one another are the number 2, and X is an anion.

2. A cyclic polyamine salt of formula (1) as claimed in claim 1, where R¹, R² and R³ are C₁-C₄-alkyl, R⁴, R⁵ and R⁶ are hydrogen, and I, m and n are 2.

3. A cyclic polyamine salt as claimed in claim 1 or 2, where R¹, R², R³, R⁴, R⁵ and R⁶ are hydrogen or methyl.

4. A cyclic polyamine salt as claimed in claim 3, where R¹, R² and R³ are methyl.

5. A cyclic polyamine salt as claimed in claims 1 to 4, where X is Cl⁻, Br⁻, I⁻, NO₃⁻, ClO₄⁻, NCS⁻, PF₆⁻, RSO₄⁻, RCOO⁻, BPh₄⁻, CF₃SO₃⁻, RSO₃⁻, R is C₁-C₄-alkyl, and Ph is phenyl.

6. A process for the preparation of cyclic polyamine salts of the formula 1 as claimed in claim 1, which comprises reacting a cyclic polyamine of the formula 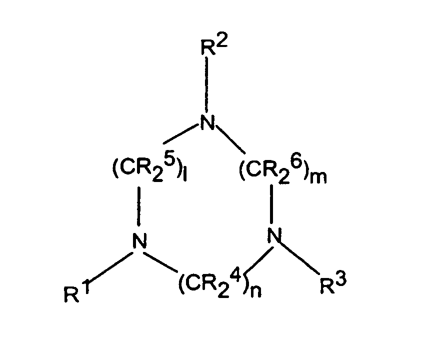 with an acid of the formula H-X, where R¹, R², R³, R⁴, R⁵, R⁶, I, m, n and X are as defined in claim 1.

7. A detergent or cleaner comprising a peroxy compound, which additionally comprises a cyclic polyamine salt of the formula 1 as claimed in claim 1.

## Revendications

1. Sels de polyamine cyclique de formule (1) 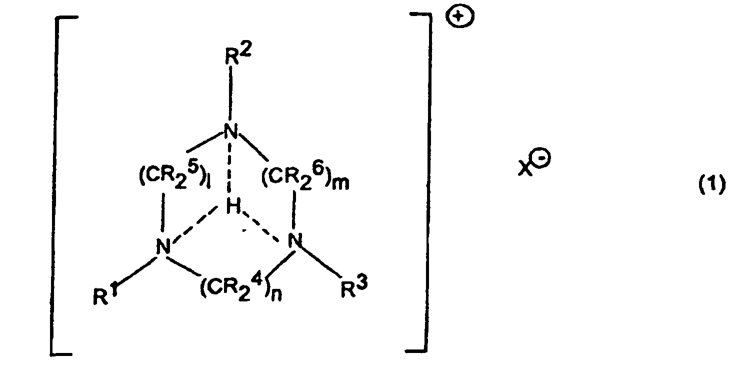 dans laquelle R¹, R², R³, R⁴, R⁵ et R⁶ représentent chacun indépendamment des autres un atome d'hydrogène ou un groupe alkyle en C₁-C₄, et 1, m et n représentent chacun indépendamment des autres le nombre 2, et X est un anion.

2. Sels de polyamine cyclique de formule (1) selon la revendication 1, dans lesquels R¹, R² et R³ sont des groupes alkyle en C₁-C₄, R⁴, R⁵ et R⁶ sont des atomes d'hydrogène, et 1, m et n valent 2.

3. Sels de polyamine cyclique selon la revendication 1 ou 2, dans lesquels R¹, R², R³, R⁴, R⁵ et R⁶ sont des atomes d'hydrogène ou des groupes méthyle.

4. Sels de polyamine cyclique selon la revendication 3, dans lesquels R¹, R² et R³ sont des groupes méthyle.

5. Sels de polyamine cyclique selon les revendications 1 à 5, dans lesquels X est Cl⁻, Br⁻, I⁻, NO₃⁻, ClO₄⁻, NCS⁻, PF₆⁻, RSO₄⁻, RCOO⁻, BPh₄⁻, CF₃SO₃⁻, RSO₃⁻, R est un groupe alkyle en C₁-C₄ et Ph est le groupe phényle.

6. Procédé de préparation de sels de polyamine cyclique de formule 1 selon la revendication 1, **caractérisé en ce qu'**on fait réagir une polyamine cyclique de formule 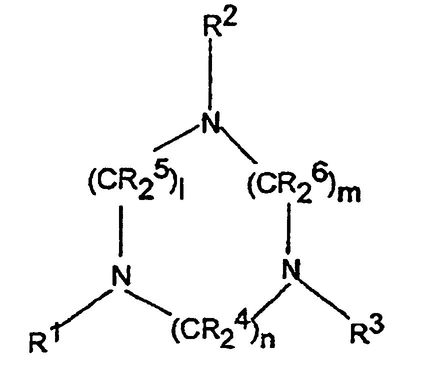 avec un acide de formule H-X, R¹, R², R³, R⁴, R⁵, R⁶, l, m, n et X ayant les significations données dans la revendication 1.

7. Produits détergents et de lavage contenant un composé peroxy, **caractérisés en ce que** le produit détergent et de lavage contient en outre un sel de polyamine cyclique de formule 1 selon la revendication 1.
